# EUROPEAN PATENT SPECIFICATION

(11) **EP 1 757 315 B1**
(45) Date of publication and mention of the grant of the patent: **02.01.2013**
(21) Application number: 05736748.4
(22) Date of filing: 28.04.2005
(51) Int. Cl.: A61L 27/00, A61L 31/00, A61C 8/00

(54) **BONE REGENERATION MEMBRANE AND IMPLANT-ATTACHED BONE REGENERATION MEMBRANE**
KNOCHENREGENERATIONSMEMBRAN UND EIN IMPLANTAT BEFESTIGT AN EINER KNOCHENREGENERATIONSMEMBRAN
MEMBRANE DE RÉGÉNERATION OSSEUSE ET UN IMPLANT ATTACHÉ À UNE MEMBRANE DE RÉGÉNERATION OSSEUSE

(30) Priority: 30.04.2004 JP 2004136725; 30.07.2004 JP 2004224148
(43) Date of publication of application: 28.02.2007
(73) Proprietor: Sunstar Suisse SA, 1024 Ecublens (CH)
(72) Inventor: NISHIMOTO, Kunio;, Osaka-shi, Osaka 5460042 (JP); KITA, Kazuyoshi, 6340813 (JP); KATSURAGI, Yasuhiro, 5650824 (JP); KATO, Hiromu, 5200815 (JP)
(74) Representative: Müller-Boré & Partner Patentanwälte
(86) International application number: PCT/JP2005/008183
(87) International publication number: WO 2005/105164

(56) References cited:
- WO-A-99/19005
- WO-A1-2004/110514
- DE-A1- 19 654 884
- JP-A- 3 505 684
- JP-A- 7 023 982
- JP-A- 9 507 144
- JP-A- 10 508 324
- JP-A- 11 507 256
- JP-A- 62 255 124
- JP-A- 2002 085 547
- JP-A- 2002 325 830
- JP-A- 2002 527 144
- JP-A- 2003 522 557
- US-B1- 6 398 814
- US-B1- 6 548 569
- KELLOMAKI M ET AL: "Bioabsorbable scaffolds for guided bone regeneration and generation" BIOMATERIALS, ELSEVIER SCIENCE PUBLISHERS BV., BARKING, GB, vol. 21, no. 24, 15 December 2000 (2000-12-15), pages 2495-2505, XP004217413 ISSN: 0142-9612

## Description

The present invention relates to bone regeneration membranes, used for regenerating bones, etc. in medical fields such as dentistry, oral surgery, orthopedics, etc.

Conventional tissue regeneration membranes, bone regeneration membranes, and like biocompatible membranes used to regenerate tissues, bones, etc. in medical fields such as dentistry, oral surgery, etc. comprise homopolymers of glycolic acid, lactic acid, caprolactone or the like, or copolymers or mixtures thereof (see Patent Document 1). Reported have been, for example, a material comprising a microporous polymer-ceramic material (see Patent Document 2); a sponge-like material comprising a polycondensate of lactic acid, glycolic acid or caprolactone, or a copolymer thereof (see Patent Document 3); a bioabsorbable membrane with pores completely penetrating therethrough produced by converting a membrane polymer material into an emulsion and then shaping (see Patent Document 4); a multilayer film comprising type II collagen (see Patent Document 5); and a membrane with a porous sheet-like structure comprising a blend of polymers selected from homopolymers and copolymers of L-lactic acid, D,L-lactic acid, glycolic acid and ε-caprolactone, the membrane having a pore size of 1 to 50 µmφ, a porosity of 5 to 95%, and a thickness of 50 to 500 µm (see Patent Document 6).

Methods using GTR (Guided Tissue Regeneration) membranes have been clinically applied to periodontal tissue deficiencies since the middle of the 1980s so as to secure a space that allows the proliferation of periodontal tissues such as alveolar bone and periodontal ligaments, while inhibiting gingival epithelial downgrowth. Such methods have some effectiveness in the regeneration of periodontal tissues such as alveolar bone. However, since most conventional GTR membranes are two-dimensional flat membranes, a tissue regeneration method using such a GTR membrane is limited in application to only specific configurations and sizes of bone defects due to the configuration, size and strength of the membrane. Therefore, a tissue regeneration membrane with a three-dimensional configuration and size more suited to an affected part has been sought. Furthermore, since conventional GTR membranes have simple configurations such as rectangular, while the space where the membrane is applied is narrow and the affected part has a complicated configuration, it is difficult to apply such a conventional GTR membrane to periodontal tissue, so that only skilled doctors have been able to utilize such membranes. To apply such membranes to bone tissue regeneration, GBR (Guided Bone Regeneration) membranes have also been developed. However, such GBR membranes find only limited application for the same reasons as for GTR membranes, so that ordinary doctors have not been able to utilize GBR membranes.

Therefore, it has been desired to develop tissue regeneration membranes and bone regeneration membranes configured to be easily applicable, the membranes being capable of securely providing a space that allows tissue regeneration in the defect, irrespective of the configuration and size of the bone defect, i.e., whether the defect is wide or in a horizontal portion, and without restriction in terms of strength of the membrane.

Conventional methods for producing biocompatible membranes such as tissue regeneration membranes and bone regeneration membranes have been reported, for example, a method of forming a film comprising dissolving a polymer material in methylene chloride to form a homogenous solution and distilling off the solvent to form a film (see Patent Document 1), and a method of producing a membrane comprising converting the starting material into an emulsion, applying the emulsion to a shape-retaining material, drying, and then peeling off the membrane from the shape-retaining material (see Patent Publication 4). However, since these methods use organic solvents, care is necessary to produce a homogenous membrane. Furthermore, to produce a membrane with a complicated configuration, the production steps are complicated.

Apart from this, selective laser sintering was developed in the 1980s and is utilized, in the fields of plastics molding and various casting technologies, to produce prototype models in the form of powder-shaped objects (used to confirm final product appearance and predict strength, etc., prior to full-scale production of final products) by using configurational data obtained by a 3-D CAD system. The material used in this method is nylon, polystyrene, polyimide, elastomer rubber, iron, stainless steel, aluminum, etc. i.e., materials having comparatively high heat resistance and being easily formed into fine particles (see Patent Documents 8 to 11). Although this inexpensive powder shaping method is effective for visually confirming three-dimensional design configurations that are difficult to illustrate in drawings, the bonding between powder particles is weak and the obtained product is fragile, thus having problems in terms of strength, durability, etc., so that this method has been rarely utilized for producing final products. In particular, since the physical structure of biocompatible materials such as polylactic acid is easily changed by heat, such materials have been considered as being unsuitable for this method. Therefore, there has been no report on utilizing such a method as a process for producing biocompatible membranes such as tissue regeneration membranes and bone regeneration membranes.
[Patent Document 1] PCT Japanese translation publication No. H06-504702
[Patent Document 2] Japanese Unexamined Patent Publication No. H06-319794
[Patent Document 3] Japanese Unexamined Patent Publication No. H10-234844
[Patent Document 4] Japanese Unexamined Patent Publication No. H11-80415
[Patent Document 5] PCT Japanese translation publication No. 2001-519210
[Patent Document 6] Japanese Unexamined Patent Publication No. 2002-85547
[Patent Document 7] PCT Japanese translation publication No. H06-504702
[Patent Document 8] PCT Japanese translation publication No. H09-511703
[Patent Document 9] PCT Japanese translation publication No. H10-505116
[Patent Document 10] PCT Japanese translation publication No. H11-509485
[Patent Document 11] PCT Japanese translation publication No. 2000-504642

JP 2002 325830 A describes a bone tissue regeneration guiding membrane, which is constituted of at least a double layer membrane, wherein the outer layer membrane is constituted of a material with a modulus of rigidity in the range of 500 to 5,000 MPa at room temperature and a glass transition temperature within the range of 40 to 60°C, and wherein the inner layer is constituted of a material with a modulus of rigidity in the range of 2.0 to 500 MPa and a glass transition temperature within the range 4 to 37°C.

DE 196 54 884 A1 describes a structure, which comprises a membrane for tissue or bone regeneration, wherein the membrane contains at least three layers; the two outer layers are made of a natural material and the inner layer(s) are made from one or more plastic materials.

WO 97/43978 describes a prosthetic apparatus for promoting and supporting guided bone tissue regeneration in at least missing or excised portions of the human mandible or maxilla and in bony defects of the mandible and maxilla.

An object of the invention is to provide bone regeneration membranes with an appropriate configuration according to the configuration of the affected part of the patient in need of bone regeneration treatment, or implant treatment.

The present inventors carried out extensive research to achieve the above object. As a general result, the inventors found that when using a lamination forming process, for example, at least one lamination forming process selected from the group consisting of (a) selective laser sintering, (b) powder adherence, (c) fused deposition molding, (d) laminated object manufacturing, and (e) optical molding, or using a powder electrostatic coating process, a homogenous biocompatible membrane with a configuration optimal for the treatment region can be produced at low cost in a short time.

More specifically, the process for producing a biocompatible membrane comprises subjecting to a biocompatible material to a lamination forming process or a powder electrostatic coating process. The process can produce a homogenous biocompatible membrane at low cost in a short time without using complicated production steps, the membrane having a size, pore configuration, porosity, thickness, etc. appropriate to the application site. The biocompatible membrane is a biodegradable membrane obtained by the above production process and can be used as a homogeneous membrane for bone or tissue regeneration treatment in the fields of dentistry (e.g., periodontal treatment), oral surgery, orthopedics, etc. In tissue regeneration, biocompatible membranes such as tissue regeneration membranes and bone regeneration membranes play roles in maintaining the existing tissue in a normal configuration, separating one tissue region from another, and further if necessary, containing a bone filler therein, etc. Such a tissue regeneration membrane is used to establish an environment that allows only the proliferation of target cells, while inhibiting the proliferation of inappropriate cells, so that the defect of the body, such as soft tissue and hard tissue, can be regenerated and restored.

In particular, in the field of dentistry, tissue regeneration membranes are used to regenerate periodontal tissue as closely to a normal state as possible. Attachment between the cementum and periodontal ligament is lost due to periodontal disease, etc. and the alveolar bone for supporting the teeth is absorbed, which causes bone tissue defects. When cell proliferation is promoted to return this state to a normal state, gingival epithelial cells and gingival connective fibroblasts proliferate at a higher rate than periodontal ligament cells (periodontal ligament fibroblasts). Consequently, gingival epithelial cells and gingival connective fibroblasts proliferate in the space where periodontal ligament fibroblasts should proliferate (root surface), thereby impeding periodontal ligament formation and suppressing the proliferation of osteoblasts. The tissue regeneration membrane invention acts as a barrier to physically inhibit other cells from entering this space, and also functions to promote the proliferation of periodontal ligament fibroblasts and periodontal ligament formation in this space and promote the proliferation of osteoblasts and formation of bone. As a result, new attachment between the hard tissue cementum and periodontal ligament and gingival connective tissue is established and alveolar bone is regenerated, so that the affected part is restored to the normal state or a state close to normal.

The tissue regeneration membrane can be applied, for example, to three-wall defects, which are comparatively mild alveolar bone defects in dentistry, one-wall defects, which are comparatively severe alveolar bone defects, furcation defects, horizontal bone defects, etc. The tissue regeneration membrane can also be applied as a scaffold for hepatic cells and growth factors such as platelet derived growth factors (PDGF), bone morphogenic proteins (BMP), fibroblast growth factors (FGFs, especially bFGFs), etc. in biomedical tissue engineering for regenerating tissues and organs with severe defects and restoring their functions, based on regenerative medicine.

In a similar manner to the tissue regeneration membrane, the bone regeneration membrane of the invention is used to establish an environment that allows only the proliferation of osteoblasts, while inhibiting the proliferation of inappropriate cells, so that the hard tissue defect can be restored and regenerated. The bone regeneration membrane of the invention is advantageously used to promote bone regeneration in dental implant treatments so that the alveolar bone can withstand implant (artificial dental implant) installment.

"Biodegradable" as used herein refers to the property of being absorbed or decomposed in the body.

The present invention provides the following membranes.

Item 1. A bone regeneration membrane for dental implant treatment, the membrane comprising an inner layer and an outer layer, the inner layer having a configuration that fits the contours of an existing periodontal tissue and periodontal tissue regeneration region, and the outer layer covering the outer surface of the inner layer with an inner space therebetween.

Item 2. A dental implant-attached bone regeneration membrane wherein a dental implant is attached to the bone regeneration membrane of item 1.

Item 3. The dental implant-attached bone regeneration membrane according to item 2 wherein the top of the dental implant is attached to the membrane.

Item 4. The dental implant-attached bone regeneration membrane according to item 2 wherein the membrane has an opening that faces the top of the dental implant.

Item 5. The bone regeneration membrane according to any one of items 1 to 4 wherein the outer layer is a bone regeneration membrane produced by a process for producing a biocompatible membrane comprising subjecting a biocompatible material to a lamination forming process or a powder electrostatic coating process, wherein the biocompatible membrane is a bone regeneration membrane.

Item 6. The dental implant-attached bone regeneration membrane according to item 5 wherein the inner layer is a bone regeneration membrane produced by a process for producing a biocompatible membrane comprising subjecting a biocompatible material to a lamination forming process or a powder electrostatic coating process, wherein the biocompatible membrane is a bone regeneration membrane.

Item 7. The bone regeneration membrane according to item 5 wherein the inner layer is produced by subjecting to a lamination forming process at least one member selected from the group consisting of calcium phosphate, α-tricalcium phosphate, β-tricalcium phosphate, tetracalcium phosphate, hydroxyapatite, and bioactive glasses.

The biocompatible material usable in the production of the bone regeneration membrane in the invention is not particularly limited. Examples of usable biocompatible materials include biodegradable resins, biocompatible inorganic materials, and derivatives thereof. Examples of biodegradable resins include homopolymers and copolymers of L-lactic acid, D-lactic acid, D,L-lactic acid, glycolic acid, ε-caprolactone, N-methylpyrrolidone, trimethylene carbonate, p-dioxanone, 1,5-dioxepan-2-one, hydroxybutanoic acid, hydroxyvaleric acid, acid anhydrides (sebacic acid anhydride, maleic acid anhydride, dioleic acid anhydride, etc.), amino acids (L-amino acids, D-amino acids, mixtures of L- and D-amino acids) such as glycine, alanine, phenylalanine, tyrosine, asparagine, glutamine, aspartic acid, glutamic acid, ricin, hydroxylysine, arginine, valine, leucine, isoleucine, serine, threonine, cysteine, methionine, tryptophan, histidine, proline, hydroxyproline, etc., and the like; mixtures of such polymers, polyesters, polycarbonates, polyacrylic acids such as poly(α-cyanoacrylate), polyphosphates, amino acid polymers, polyacid anhydrides, proteins (gelatin, collagen, etc.), polyglycosides (chitin, chitosan, starch, etc.), etc. Examples of biocompatible inorganic materials include calcium phosphates (calcium phosphate, α-tricalcium phosphate, β-tricalcium phosphate, tetracalcium phosphate, hydroxyapatite), bioactive glasses, etc. Among biodegradable resins, homopolymers and copolymers of at least one compound selected from the group consisting of lactic acid, glycolic acid and ε-caprolactone, and mixtures of such polymers, are preferable, and polylactic acid is particularly preferable. Such biodegradable resins generally have an average molecular weight of 2000 to 2000000, and preferably 20000 to 500000, and preferably have a mean particle diameter of 0.001 to 0.3 mm, and most preferably 0.001 to 0.1 mm. Biocompatible materials with such a mean particle diameter can be obtained by, for example, pulverizing a conventional biocompatible material at a low temperature of -120°C or less, or dispersing the material in water or a solvent and removing the solvent.

In addition to such biocompatible materials, the use of biodegradable plasticizers such as citrate esters such as acetyl tri-n-butyl citrate, triethyl citrate, etc., maleic acid esters, adipic acid esters, etc. can improve formability and provides a membrane that is malleable to the tissue at the time of implantation and adapts itself to movement and displacement of the tissue after implantation without damaging the tissue. The proportion of biodegradable plasticizer in the biocompatible material may be 3 to 30 wt.%. A proportion within the above-mentioned range is advantageous in terms of formability. Such a biodegradable plasticizer can, for example, be mixed with the biocompatible material and the resulting powder formed into a membrane according to a powder forming process.

The biocompatible membrane may further comprise appropriate pharmaceutical agents. Examples of such pharmaceutical agents include bone and tissue growth factors; tissue-derived substances such as albumin, globulin, chondroitin sulfate, fibronectin, fibrinogen and elastin; antibiotics, for example, tetracyclines such as minocycline and doxycycline, macrolides such as clarithromycin and azithromycin, new quinolones such as levofloxacin, and ketolides such as telithromycin; antiinflammatory agents, for example, non-steroidal anti-inflammatory drugs such as flurbiprofen, and steroids such as dexamethasone; naturally derived substances such as azulene; bone-absorption inhibitors such as bisphosphonate; etc. Such pharmaceutical agents can be placed on the inside of the inner layer of the membrane of the invention by, for example, coating or filling in the form of gels.

The biocompatible membrane can be produced, for example, by a process comprising obtaining three-dimensional information on the configuration of an application site (e.g., affected part) to which a biocompatible membrane such as a tissue regeneration membrane or a bone regeneration membrane is to be applied in dental treatment by a method such as radiation tomography such as CT scanning or nuclear magnetic resonance imaging (MRI), and forming a biocompatible membrane with a configuration suitable for the treatment, based on the three-dimensional information, by a lamination forming process such as (a) selective laser sintering, (b) powder adherence, (c) fused deposition molding, (d) laminated object manufacturing, or (e) optical molding.
(a) Examples of selective laser sintering processes that can be used include three-dimensional powder forming processes (powder sintering processes using lasers). More specifically, according to such a process, a pre-prepared biocompatible material powder with a mean particle diameter of 0.001 to 0.3 mm is placed to a predetermined thickness at predetermined position(s) on a support, based on the dimensional information of a 3-D CAD model pre-prepared by CAD, etc. If necessary, the surface of the powder may be smoothed using a wiper, etc. Subsequently, to form a configuration corresponding to the model, the biocompatible material powder is irradiated with a laser such as a carbon dioxide laser, fused, and solidified. This procedure is repeated with the support being shifted in the vertical direction, thus forming an object corresponding to the model.
   Lasers used in selective laser sintering are not particularly limited. Examples of usable lasers include infrared lasers, carbon dioxide lasers, YAG and like solid lasers, excimer lasers, etc. The output power and beam width of such a laser are suitably selected according to the biocompatible material used, shape of the object, etc.
(b) Examples of powder adherence processes include a process applying an ink-jet technique as used in printers, etc. (deposition process), which comprises continuously depositing thermally fused wax or like droplets and solidifying the droplets; a lamination forming process (binder process) comprising ejecting a binder from an ink-jet head onto a metal powder, ceramic powder, starch powder, or gypsum powder to adhere the powder; a process (photocuring process) comprising ejecting a resin powder from an ink-jet head and then curing by light. For example, one useful process comprises preparing several cross-sectional slices of an affected part by radiation tomography such as CT scanning, nuclear magnetic resonance imaging (MRI), etc., spraying a fixation agent (for example, collagen, chondroitin sulfate, hyaluronic acid, elastin, etc.) over a thin (e.g., about 0.1 µm) layer of biocompatible resin powder on the sheet by an ink-jet technique as used in printers, etc. in accordance with a cross-sectional slice so as to fix the biocompatible resin powder to the sheet, repeating this procedure according to each cross-sectional slice; and layering the obtained sheets, thereby providing a biocompatible membrane with the desired configuration.
(c) Examples of fused deposition molding processes include a process comprising determining the configuration of the membrane to be produced, based on the information on an affected part obtained by radiation tomograpy such as CT scanning and nuclear magnetic resonance imaging (MRI), continuously ejecting (for example, linearly ejecting) a thermoplastic resin (e.g., polycarbonate resin, etc.) in a molten state from a nozzle by heating and melting while scanning using a XY plotter system, extruding the melt, and solidifying to form a layer on the surface. In this process, the resin particles adhere to each other before curing.
(d) Examples of laminated object manufacturing processes include a process comprising determining the configuration of the membrane to be produced, based on the information on the affected part, in a manner similar to fused deposition molding (c), subjecting adhesive agent-coated material sheets to compression bonding (e.g., thermocompression bonding) using rollers, etc., excising the unnecessary portions along the contours by means of a laser, knife, etc., and repeating this procedure to form a membrane with the desired configuration.
(e) Examples of optical molding processes include a process of producing a three-dimensional membrane comprising curing and layering one liquid photocurable resin layer after another by laser or like optical beams to form a laminate. This molding process can easily form even complicated configurations and produce membranes with high dimensional accuracy.

According to the powder electrostatic coating process, the configuration of the biocompatible membrane to be produced is determined, based on the information on the affected part, and a "negative" mold of this configuration is prepared. Using the "negative" mold as a substrate to be coated by electrostatic coating, an atomized negatively charged powder is applied to the substrate, which acts as the opposite pole, by a spray gun, etc., thus producing a biocompatible membrane.

Such forming processes and powder electrostatic coating processes provide multilayer membranes with void spaces between the layers. It is also possible to form specifically-shaped pores penetrating through such objects and form specific configurations such as convex and concave regions on the inner and/or outer surface of the objects, thus providing an optimal biocompatible membrane according to the purpose and use of the membrane.

The biocompatible membrane is characterized by being produced by one of the above production processes. Since the biocompatible membrane is produced by any one of the above production processes suitable for three-dimensional shaping, the membrane can be formed into an appropriate configuration according to the configuration of the affected part of the patient.

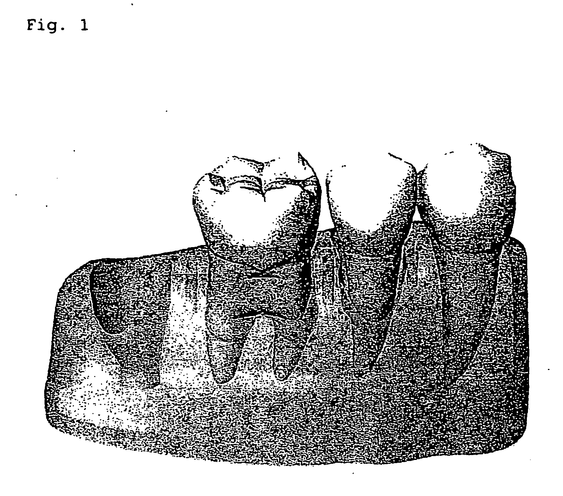

The relationship between alveolar bone and teeth in periodontal treatment is described with reference to Fig. 1. Fig. 1 is a schematic view showing the relationship between alveolar bone and teeth. The portion that looks like a foundation is alveolar bone. Although other periodontal tissues such as gingival epithelia, connective tissues and periodontal ligaments exist in addition to the alveolar bone, only the alveolar bone and teeth are shown herein to facilitate the description of the invention. In Fig. 1, the most right-hand tooth has alveolar bone in a healthy condition such that the alveolar bone entirely covers the root. The second and third teeth from the right have alveolar bone that covers only the lower half of the root, and the upper half of the alveolar bone is lacking. Since the tooth has mobility in this condition, it is necessary to reinforce the alveolar bone. Conventionally, a rectangular, circular, elliptical or like shaped membrane is cut into a suitable shape by the practitioner and the alveolar bone defect is covered with the cut membrane (e.g., GTR membrane). To prevent the membrane from moving from the defect, a ligature attached to the membrane is tied around the tooth to immobilize the membrane. However, it is difficult to apply such a conventional flat membrane by fixing the membrane in an appropriate position of the defect, and it is also very difficult to tie the ligature to the tooth by this method, thus being problematic in that only skilled operators can do this quickly. Furthermore, the membrane applied to posterior teeth cannot be firmly anchored by this fixation method, thus have the problem of insufficient fixation.

Since the tissue regeneration membrane can be formed into a complicated three-dimensional shape according to the configuration of the periodontal tissue defect, the membrane can secure a space for periodontal tissue regeneration better than conventional membranes, so that the necessity of immobilizing the membrane using a ligature, etc. is reduced and even non-skilled operators can operate quickly.

Furthermore, the most left-hand alveolar bone of Fig. 1 shows alveolar bone in need of implant treatment. There is no root, hence requiring implant treatment, and the height of the alveolar bone is also insufficient as a result of alveolar bone defects. For implant treatment, the alveolar bone needs to be regenerated appropriately to fix the dental implant to the alveolar bone defect. GBR is a method for regenerating alveolar bone. Bone regeneration methods before implant operation are roughly classified into two-step methods and one-step methods. In two-step methods, after the bone regeneration necessary for implantation has been established, a dental implant (also called "fixture") is implanted. In one-step methods, the dental implant is implanted simultaneously with GBR. In one-step methods, the bottom of the dental implant is embedded about 1 to 3 mm into the residual alveolar bone and the alveolar bone defect is filled with a bone filler to fix the dental implant (for example, Japanese Unexamined Patent Publication No. H07-23982). Conventional methods attempt to prevent epithelial tissue from entering the alveolar bone defect and secure the alveolar bone regeneration region by covering the alveolar bone defect with a rectangular, elliptical or like shaped membrane made of polytetrafluoroethylene, etc. However, when the alveolar bone defect is large, fixing the membrane to the alveolar bone by using metal pins causes membrane collapse. In implant treatment, since X-ray information on the position and direction of the dental implant is only two-dimensional, it is difficult to understand the position three-dimensionally, so that operators have to rely on their experience.

Since the bone regeneration membrane of the invention can be formed into a complicated three-dimensional shape according to the shape of the alveolar bone, the membrane can secure a space for alveolar bone regeneration better than conventional GBR membranes, so that the necessity of anchoring the membrane using pins, etc. is reduced. In particular, when using a dental implant-attached bone regeneration membrane, the position and direction of the implant are determined in advance according to the affected part and bone is formed accordingly. Therefore, the number of operational failures is reduced and the operation can be accomplished regardless of the skill of the operator. The dental implant-attached bone regeneration membrane of the invention is advantageously used in one-step methods, and the bone regeneration membrane of the invention without a dental implant is advantageously used in two-step methods.

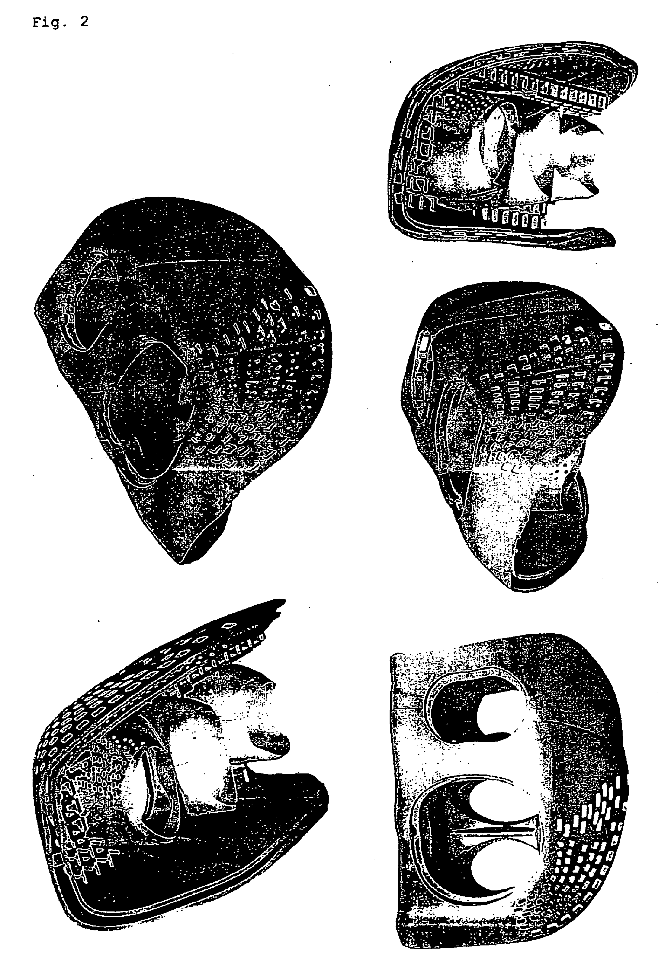

Fig. 2 is schematic views showing one reference embodiment of the tissue regeneration membrane from various angles. The membrane comprises the inner and outer layers. The inner layer has a configuration that fit the contours of the residual alveolar tissue and the periodontal tissue regeneration region. The material of the outer layer is preferably biodegradable resin(s), and the outer layer covers the outer surface of the inner layer with an inner space therebetween. The material of the inner layer is biocompatible material(s), which may be biodegradable resin(s) or biocompatible inorganic material(s) such as calcium phosphates (e.g., calcium phosphate, α-tricalcium phosphate, β-tricalcium phosphate, tetracalcium phosphate, hydroxyapatite), and bioactive glasses. Such a biodegradable resin is gradually absorbed into the body, and the biocompatible inorganic material is gradually integrated with the bone tissue. Therefore, it is unnecessary to remove the membrane after periodontal tissue regeneration. The inner layer usually has a thickness of 0.01 to 0.2 mm, and the outer layer usually has a thickness of 0.01 to 0.2 mm. The total membrane thickness is usually 0.2 to 0.7 mm. In Fig. 2, cylindrical spacers are shown on the inner surface of the inner layer. The spacers are effective for maintaining the shape and strength of the membrane. In Fig. 2, the outer layer does not entirely cover the inner layer. This is because part of the outer layer is not shown in order to more clearly show spacers provided on and holes in the inner layer. The outer layer may cover the entire inner layer. Fig. 2 shows only holes provided in the left front portion of the inner layer. This is because holes provided in the other portions of the inner layer are not shown in order to more clearly show the spacers provided on the inner layer. Preferably, the holes are provided uniformly in the entire inner and outer layers.

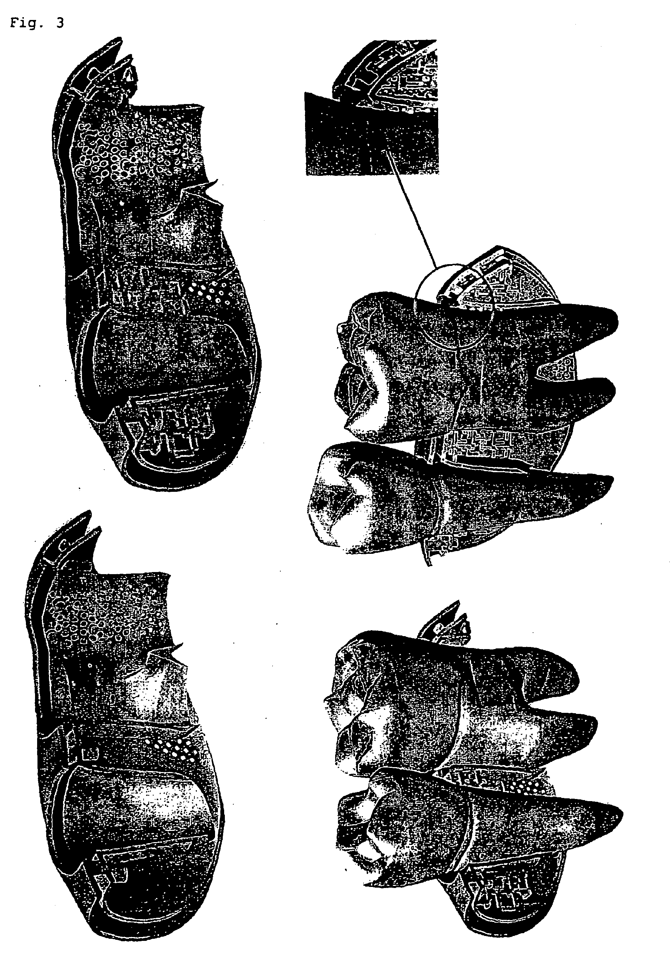

The upper portion of Fig. 3 is a schematic sectional view of the tissue regeneration membrane show in Fig. 2. The lower portion of Fig. 3 is a schematic sectional view illustrating the application of the membrane to the affected part. To clearly show the structure of the membrane, periodontal tissues such as alveolar bone are not shown in the lower section of Fig. 3. In periodontal tissue regeneration treatment, when the membrane shown in Fig. 2 whose inner layer holes in the region along the root are smaller than the teeth crowns is applied to the affected part by covering the teeth with the membrane from the above, application of the membrane may be impossible as is. Therefore, the membrane shown in Fig. 2 is divided into a membrane as shown in the upper portion of Fig. 3 and another counterpart membrane (not shown) and can be applied by sandwiching the tooth between the two membranes from both sides. Thus a membrane suitable for application can be formed by preparing a membrane with a configuration that fits the entire affected part (for example, the membrane shown in Fig. 2) and then dividing the membrane into portions suitable for application to the affected part. It is also possible to separately produce membranes suitable for application (for example, the membrane shown in the upper section of Fig. 3 and the counterpart membrane not shown therein). All such membranes are included within the scope of the tissue regeneration membrane.

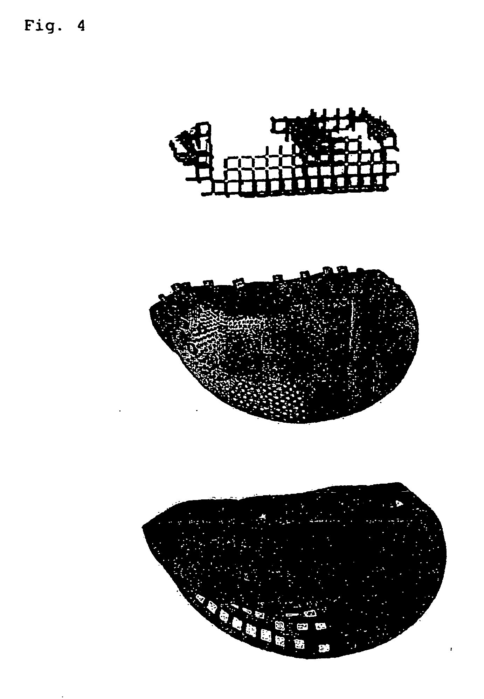

Fig. 4 is schematic views showing the structure of the tissue regeneration membrane shown in Fig. 2. The upper portion of Fig. 4 shows a support structure interposed between alveolar bone and the inner layer of the tissue regeneration membrane. The support structure is optical and not essential in the tissue regeneration membrane. The support structure can be produced using a biodegradable resin, biocompatible inorganic material, etc. according to a lamination forming process or a powder electrostatic coating process, simultaneously with the production of the tissue regeneration membrane. Such a support structure plays roles in improving the membrane strength and providing space for regeneration of periodontal tissues such as alveolar bone and periodontal ligament between the bone defect and the inner layer. When the support structure is made of a biocompatible inorganic material, it further plays a role in bone induction, etc.

The central portion of Fig. 4 shows the inner layer of the tissue regeneration membrane, and the lower portion of Fig. 4 shows the outer layer of the tissue regeneration membrane. In Fig. 4, although cylindrical spacers for securing the space between the inner and outer layers of the membrane are shown on the outer surface of the inner layer, the spacers may be provided on the inner surface of the outer layer. Such cylindrical spacers are preferably made of a biodegradable resin. The configuration of the spacers is not limited to cylindrical. The gap between the inner layer and the outer layer is usually 0.01 to 2 mm, and preferably 0.1 to 0.2 mm.

Although Figs. 2 to 4 illustrate a tissue regeneration membrane with a configuration that entirely surrounds the root, the membrane does not necessarily have to surround the entire root but may be formed into a suitable configuration depending on the condition of periodontal defect and regeneration area.

The outer and inner layers of a suitable bone regeneration membrane are provided with holes. By providing such holes, factors (e.g., bone growth factors) necessary for interaction between the epithelial tissue and bone cells can pass therethrough, thus being advantageous to bone regeneration. In the Figs., although the inner and outer layers have circular or rectangular through-holes, the shape of such holes is not limited thereto. The diameter or side length of such holes is usually 0.02 to 2 mm. Two or more types of holes that differ in shape and size may be used in combination. The inner layer preferably has circular pores with a diameter of 0.05 to 0.5 mm. The outer layer preferably has rectangular holes with a side length of 0.05 to 2 mm. Rectangular holes are preferably formed in such a manner that when the membrane is disposed in the periodontal tissue treatment region, the long axis of each hole is horizontal and the short axis is perpendicular. The holes in the outer layer are preferably larger in area than those in the inner layer.

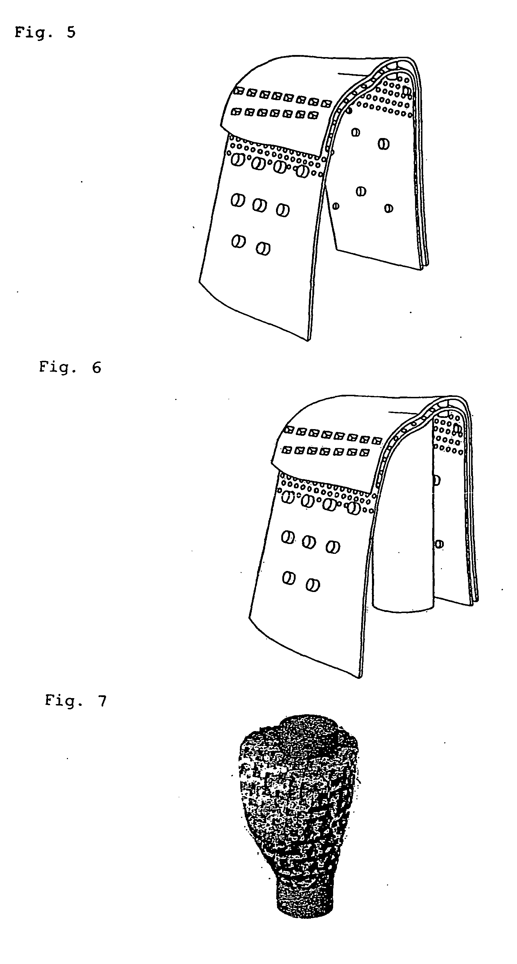

Fig. 5 is a schematic view of a bone regeneration membrane of the invention. The membrane comprises an inner layer and an outer layer, the inner layer having a configuration that fits the contours of the residual alveolar bone and the alveolar bone regeneration region, and the outer layer preferably being made of a biodegradable resin and covering the outer surface of the inner layer with an inner space therebetween. The inner layer is made of a biocompatible material, which may be a biodegradable material or a biocompatible inorganic material such as calcium phosphate (calcium phosphate, α-tricalcium phosphate, β-tricalcium phosphate, tetracalcium phosphate, hydroxyapatite), bioactive glass, etc. The biodegradable resin is gradually absorbed into the body and the biocompatible inorganic material is gradually integrated with the bone tissue. Therefore, it is unnecessary to remove the membrane after alveolar bone regeneration. The inner layer usually has a thickness of 0.01 to 0.2 mm, and the outer layer usually has a thickness of 0.01 to 0.2 mm. The total membrane thickness is usually 0.2 to 0.7 mm. In Fig. 5, although cylindrical spacers between the inner layer and the outer layer are shown on the outer surface of the inner layer, the spacers may be provided on the inner surface of the outer layer. The spacers are effective for maintaining the shape and strength of the membrane. The cylindrical spacers are preferably made of a biodegradable resin. The outer and inner layers of the bone regeneration membrane are preferably provided with holes. By providing such holes, factors (e.g., bone growth factors) necessary for interaction between the epithelial tissue and bone cell can pass therethrough, thus being advantageous to bone regeneration. In Fig. 5, although the inner and outer layers have circular or rectangular through-holes, the shape of the holes is not limited thereto. The diameter or side length of the hole is usually 0.02 to 2 mm. Two or more types of holes that differ in shape and size may be used in combination. The inner layer preferably has circular pores with a diameter of 0.05 to 0.5 mm. The outer layer preferably has rectangular holes with a side length of 0.05 to 2 mm. The rectangular holes are preferably formed in such a manner that when the membrane is disposed in the implant treatment region, the long axis of each hole is horizontal and the short axis is perpendicular. The holes in the outer layer preferably are larger in area than those in the inner layer. In Fig. 5, cylindrical spacers are also shown on the inner surface of the inner layer. This is effective for maintaining the shape and strength of the membrane. In Fig. 5, the outer layer does not entirely cover the outer surface of the inner layer. This is because part of the outer layer is not shown so as to more clearly show the spacers and holes provided in the inner layer. In Fig. 5, the holes formed in the inner layer are shown only for the upper portion. This is because the holes provided in the lower portion of the inner layer are not shown so as to more clearly show the spacers provided on the inner layer. Preferably, the holes of the inner and outer layers are provided uniformly in the upper to lower portions.

Fig. 6 is a schematic view of a dental implant-attached bone regeneration membrane of the invention. A dental implant is attached to a membrane having the same configuration as shown in Fig. 5. The dental implant may be one known for use in implantation, and is preferably made of titanium or a titanium alloy highly compatible with bone cells. The configuration of the dental implant may be a known configuration. The dental implant is securely fixed by attaching the top of the dental implant to the membrane of the invention and fixing the bottom of the dental implant to the residual alveolar bone. To more firmly fix the implant, known bone fillers (e.g. hydroxyapatite, α-TCP (tricalcium phosphate), β-TCP, tetracalcium phosphate, calcium hydrogenphosphate) may be used to fill in the alveolar bone regeneration region except for where the dental implant is positioned, or a dental implant with a support structure that conforms to the configuration of the alveolar bone regeneration region (Fig. 7) may be utilized. Such a support structure can be prepared using biodegradable resins, biocompatible inorganic materials, etc. by a lamination forming process or a powder electrostatic coating process, simultaneously with the production of the dental implant-attached bone regeneration membrane.

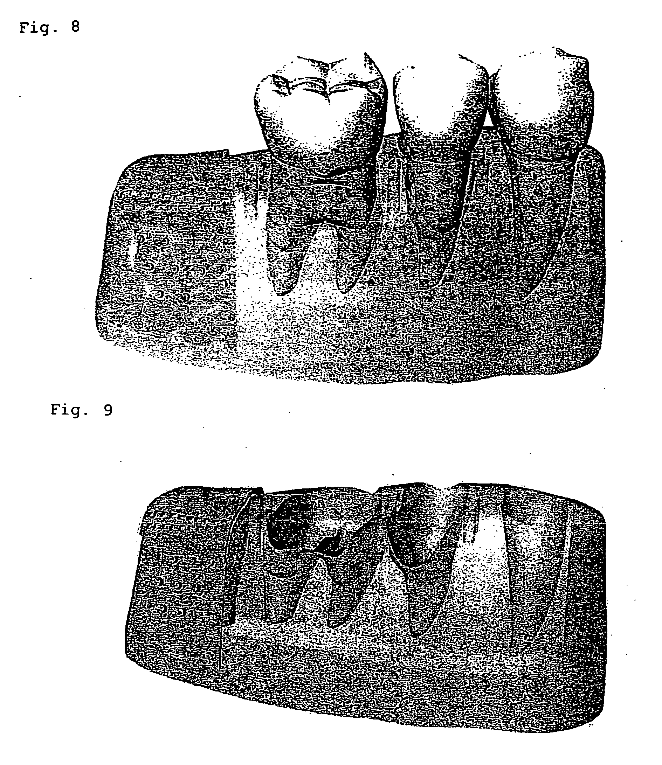

Fig. 8 is a schematic view illustrating the application of the bone regeneration membrane shown in Fig. 5 to alveolar bone. Since the membrane is formed into a desired shape according to the patient's affected part, in particular, the state of alveolar bone, as determined by CT scanning, MRI, etc., the membrane fits the configuration of the alveolar bone more accurately than conventional membranes. In this case, the dental implant is implanted after hard tissue for the dental implant has been regenerated.

Fig. 9 is a schematic view illustrating the application of the dental implant-attached bone regeneration membrane provided with a support structure as shown in Fig. 7. As with the membrane shown in Fig. 8, this membrane more precisely fits the configuration of the alveolar bone than conventional membranes, and firmer fixation can be achieved than with standard dental implant fixtures.

A lamination forming process or a powder electrostatic coating process may be used to produce biocompatible membranes such as tissue regeneration membranes, bone regeneration membrane, etc. with an appropriate size, pore configuration, porosity, thickness, etc. according to the application site, at low cost in a short time, without using any complicated production steps. The biocompatible membranes are produced by the above production processes, have a size, pore shape, porosity and thickness that are more appropriate for the application site than conventional membranes, and can be utilized in tissue regeneration treatment in the fields of dental treatment, oral surgery, etc. In particular, the dental implant-attached bone regeneration membrane of the present invention can be used in one-step methods to more firmly fix the dental implant.

Since the powder electrostatic coating process utilizes a negative mold to produce the membrane, a product with high dimensional accuracy can be obtained and the method is suited to GBR membranes. Such a GBR membrane can be used as a bone regeneration membrane comprising an inner layer and an outer layer, the inner layer having a configuration that fits the contours of the residual alveolar bone and the alveolar bone regeneration region, and the outer layer covering the outer surface of the inner layer with an inner space between the inner and outer layers. An artificial dental implant for implant treatment may be attached to the bone regeneration membrane.

Fig. 1 is a schematic view illustrating alveolar bone in need of implant treatment, alveolar bone and tooth in need of periodontal tissue regeneration treatment, and healthy alveolar bone and tooth.

Fig. 2 is a schematic view of a tissue regeneration membrane.

Fig. 3 is a sectional view of the tissue regeneration membrane shown in Fig. 2 (upper portion), and a schematic view of the application of the membrane to the affected part (lower portion).

Fig. 4 is a schematic view of the support structure (upper portion), inner layer (central portion), and the outer layer (lower portion) of the tissue regeneration membrane shown in Fig. 2.

Fig. 5 is a schematic view of a bone regeneration membrane of the invention.

Fig. 6 is a schematic view of a dental implant-attached bone regeneration membrane.

Fig. 7 is a schematic view of a dental implant provided with a support structure that matches the configuration of an alveolar bone regeneration region.

Fig. 8 is a schematic view illustrating the application of the bone regeneration membrane shown in Fig. 5 to alveolar bone.

Fig. 9 is a schematic view illustrating the application of a dental implant-attached bone regeneration membrane provided with the support structure shown in Fig. 7 to the alveolar bone.

Examples are given below to describe the invention in more detail. However, the invention is not limited thereto or thereby.

### EXAMPLES

### Example 1 (reference)

A fine powder (mean particle diameter: 100 µm) was obtained by emulsifying polylactic acid and removing the solvent. The target region for periodontal tissue regeneration treatment was subjected to CT scanning to obtain three-dimensional digital data of the treatment region. Based on the data, the design of a configuration suitable for the treatment was prepared, then subjected to data processing and used as three-dimensional lamination forming data. The above-obtained polylactic powder was placed in a powder lamination forming machine. More specifically, using a movable container, the fine powder in an amount appropriate for a specific thickness was supplied to the worktable of the machine at 155°C in a flat manner, and the fine powder was irradiated with a carbon dioxide laser (50W) according to cross-sectional slices of the 3-dimensional configuration to fix the specific thickness of the fine powder by sintering or fusion. By repeating this procedure and removing excess fine powder using a brush and a suitable solvent such as ethanol, a desired periodontal tissue regeneration membrane was provided with a configuration suited to the periodontal tissue regeneration treatment region. Thus a membrane for periodontal tissue regeneration treatment with a configuration conforming to the treatment region was obtained (Figs. 2 and 3).

### Example 2 (reference)

The target region for periodontal tissue regeneration treatment was subjected to CT scanning to prepare three-dimensional digital data of the treatment region. Based on this data, two differently shaped models that fit the treatment region, i.e., an inner layer model with circular holes and cylindrical spacers, and an outer layer model with rectangular holes, were prepared using appropriate resins. A polylactic acid fine powder was electrostatically applied thereto and heated to provide a tissue regeneration membrane.

### Example 3

A fine powder (mean particle diameter: 100 µm) was obtained by emulsifying polylactic acid and removing the solvent. The target region for implant treatment was subjected to CT scanning to obtain three-dimensional digital data of the treatment region. Based on the data, the design of a configuration suitable for the treatment was prepared, then subjected to data processing and used as three-dimensional lamination forming data. The above-obtained polylactic powder was placed in a powder lamination forming machine. More specifically, using a movable container, the fine powder in an amount appropriate for a specific thickness was supplied to the worktable of the machine at 155°C in a flat manner, and the fine powder was irradiated with a carbon dioxide laser (50W) according to cross-sectional slices of the 3-dimensional configuration to fix the specific thickness of the fine powder by sintering or fusion. By repeating this procedure and removing excess fine powder using a brush and a suitable solvent such as ethanol, a desired bone tissue regeneration membrane was provided with a configuration suited to the implant treatment region. Further, to mechanically mate this membrane with the grooves or ribs formed on the dental implant, the ribs formed on the three-dimensional regeneration membrane were engaged and mated to integrate the three-dimensional tissue regeneration membrane with the dental implant. Thus a bone regeneration membrane for implant treatment with a configuration conforming to the treatment region was obtained (Fig. 6).

The present invention is useful for bone regeneration membranes used for regenerating bones, etc. in medical fields such as dentistry, oral surgery, orthopedics, etc.

## Claims

1. A bone regeneration membrane for dental implant treatment, the membrane comprising an inner layer and an outer layer, the inner layer having a configuration that fits the contours of an existing periodontal tissue and periodontal tissue regeneration region, and the outer layer covering the outer surface of the inner layer with an inner space therebetween.

2. A dental implant-attached bone regeneration membrane wherein a dental implant is attached to the bone regeneration membrane of claim 1.

3. The dental implant-attached bone regeneration membrane according to claim 2 wherein the top of the dental implant is attached to the membrane.

4. The dental implant-attached bone regeneration membrane according to claim 2 wherein the membrane has an opening that faces the top of the dental implant.

5. The bone regeneration membrane according to any one of claims 1 to 4 wherein the outer layer is a bone regeneration membrane produced by a process for producing a biocompatible membrane comprising subjecting a biocompatible material to a lamination forming process or a powder electrostatic coating process, wherein the biocompatible membrane is a bone regeneration membrane.

6. The dental implant-attached bone regeneration membrane according to claim 5 wherein the inner layer is a bone regeneration membrane produced by a process for producing a biocompatible membrane comprising subjecting a biocompatible material to a lamination forming process or a powder electrostatic coating process, wherein the biocompatible membrane is a bone regeneration membrane.

7. The bone regeneration membrane according to claim 5 wherein the inner layer is produced by subjecting to a lamination forming process at least one member selected from the group consisting of calcium phosphate, α-tricalcium phosphate, β-tricalcium phosphate, tetracalcium phosphate, hydroxyapatite, and bioactive glasses.

## Patentansprüche

1. Knochenregenerationsmembran zur Zahnimplantatbehandlung, wobei die Membran eine innere Schicht und eine äußere Schicht umfasst, wobei die innere Schicht eine Konfiguration aufweist, die zu den Konturen eines bestehenden periodontalen Gewebes und periodontalen Geweberegenerationsbereichs passt, und die äußere Schicht die äußere Oberfläche der inneren Schicht mit einem inneren Abstand dazwischen bedeckt.

2. An einem Zahnimplantat befestigte Knochenregenerationsmembran, wobei ein Zahnimplantat an der Knochenregenerationsmembran nach Anspruch 1 befestigt ist.

3. An einem Zahnimplantat befestigte Knochenregenerationsmembran nach Anspruch 2, wobei das obere Ende des Zahnimplantats an der Membran befestigt ist.

4. An einem Zahnimplantat befestigte Knochenregenerationsmembran nach Anspruch 2, wobei die Membran eine Öffnung aufweist, die dem oberen Ende des Zahnimplantats zugewandt ist.

5. Knochenregenerationsmembran nach einem der Ansprüche 1 bis 4, wobei die äußere Schicht eine Knochenregenerationsmembran ist, hergestellt durch ein Verfahren zur Herstellung einer biokompatiblen Membran, umfassend das Unterziehen eines biokompatiblen Materials einem Laminierungsformverfahren oder einem elektrostatischen Pulverbeschichtungsverfahren, wobei die biokompatible Membran eine Knochenregenerationsmembran ist.

6. An einem Zahnimplantat befestigte Knochenregenerationsmembran nach Anspruch 2, wobei die innere Schicht eine Knochenregenerationsmembran ist, hergestellt durch ein Verfahren zur Herstellung einer biokompatiblen Membran, umfassend das Unterziehen eines biokompatiblen Materials einem Laminierungsformverfahren oder einem elektrostatischen Pulverbeschichtungsverfahren, wobei die biokompatible Membran eine Knochenregenerationsmembran ist.

7. Knochenregenerationsmembran nach Anspruch 5, wobei die innere Schicht durch das Unterziehen mindestens eines Mitglieds, ausgewählt aus der Gruppe, bestehend aus Calciumphosphat, α-Tricalciumphosphat, β-Tricalciumphosphat, Tetracalciumphosphat, Hydroxylapatit, und bioaktiven Gläsern, einem Laminierungsformverfahren hergestellt wird.

## Revendications

1. Membrane de régénération osseuse pour un traitement d'implant dentaire, la membrane comprenant une couche intérieure et une couche extérieure, la couche intérieure ayant une configuration qui s'adapte aux contours d'un tissu périodontique existant et d'une zone de régénération de tissu périodontique, et la couche extérieure couvrant la surface externe de la couche intérieure avec un espace interne entre elles.

2. Membrane de régénération osseuse fixée à l'implant dentaire, dans laquelle un implant dentaire est fixé à la membrane de régénération osseuse selon la revendication 1.

3. Membrane de régénération osseuse fixée à l'implant dentaire selon la revendication 2, dans laquelle le haut de l'implant dentaire est fixé à la membrane.

4. Membrane de régénération osseuse fixée à un implant dentaire selon la revendication 2, dans laquelle la membrane a une ouverture qui est orientée vers le haut de l'implant dentaire.

5. Membrane de régénération osseuse selon l'une quelconque des revendications 1 à 4, dans laquelle la couche extérieure est une membrane de régénération osseuse produite par un procédé visant à produire une membrane biocompatible, comprenant la soumission d'un matériau biocompatible à un procédé de formation de stratification ou un procédé de revêtement électrostatique pulvérulent, dans lequel la membrane biocompatible est une membrane de régénération osseuse.

6. Membrane de régénération osseuse fixée à l'implant dentaire selon la revendication 5, dans laquelle la couche intérieure est une membrane de régénération osseuse produite par un procédé visant à produire une membrane biocompatible, comprenant la soumission d'un matériau biocompatible à un procédé de formation de stratification ou un procédé de revêtement électrostatique pulvérulent, dans laquelle la membrane biocompatible est une membrane de régénérescence osseuse.

7. Membrane de régénération osseuse selon la revendication 5, dans laquelle la couche intérieure est produite en soumettant à un procédé de formation de stratification au moins un élément choisi dans le groupe constitué du phosphate de calcium, du phosphate α-tricalcique, du phosphate tétracalcique, de l'hydroxyapatite et des verres bioactifs.
